(19) Europäisches Patentamt
European Patent Office
Office européen des brevets

(11) **EP 3 298 916 A2**

(12) **EUROPEAN PATENT APPLICATION**

(43) Date of publication:
**28.03.2018 Bulletin 2018/13**

(51) Int Cl.:
***A24F 47/00*** *(2006.01)*

(21) Application number: **18151624.6**

(22) Date of filing: **15.01.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(30) Priority: **17.01.2017 CN 201710034320**

(71) Applicant: **Shenzhen First Union Technology Co., Ltd.**
**Shenzhen Guangdong (CN)**

(72) Inventors:
• **ZHAO, Xiaoqiang**
  **Shenzhen, Guangdong 518100 (CN)**
• **YAN, Huiyong**
  **Shenzhen, Guangdong 518100 (CN)**
• **XU, Zhongli**
  **Shenzhen, Guangdong 518100 (CN)**
• **LI, Yonghai**
  **Shenzhen, Guangdong 1-3F, Building (CN)**

(74) Representative: **ZHAOffice SPRL**
**Rue de Bedauwe 13**
**5030 Gembloux (BE)**

(54) **ELECTRONIC CIGARETTE, ATOMIZER AND CONTROL METHOD THEREOF**

(57)    An electronic cigarette, an atomizer and control method thereof are provided. The atomizer includes a housing (11, 21) defining a gas passage (15, 25) and an accommodation space (16, 26) communicated with the gas passage (11, 21), an atomizing unit (12) arranged in the accommodation space (16, 26) and configured to generate aerosol passing through the gas passage (15, 25) for user, a detecting unit (13, 23) arranged in the gas passage (15, 25) and configured to detect a gas flow quantity in the gas passage (15, 25) and a control unit (14) electrically connected to the detecting unit (13, 23) and configured to adjust an atomizing quantity of the atomizer according to the gas flow quantity detected by the detecting unit (15, 25).

FIG. 2

Description

CROSS REFERENCE TO RELATED APPLICATIONS

This application claims priority to Chinese Patent Application No. 201710034320.2 filed on January 17, 2017, which are hereby incorporated by reference herein as if set forth in its entirety.

TECHNICAL FIELD

The present disclosure generally relates to electronic cigarettes, and more particular relates to an electronic cigarette, an atomizer and a method for controlling the atomizer.

BACKGROUND

As an aerosol generator, electronic cigarettes atomize tobacco liquid to form aerosol for user. When the tobacco liquid is made, tar and other harmful substances are removed, which would eliminate the harm to human.

How to use an electronic cigarette correctly is a difficult problem for ordinary consumers. When the electronic cigarette is used under an excessive power, the atomizing quantity would be excessive, which is not only harmful to human, but also easy to damage an atomizer of the electronic cigarette. While the electronic cigarette is used under a too small power, it would not satisfy users' requirement.

At present, a temperature control electronic cigarette could realize the purpose of healthy smoking by controlling the temperature of the heating body in the electronic cigarette. However, the temperature control electronic cigarette has disadvantages of having a complex temperature control system, needing certain requirements for the heating body, and having low temperature control precision.

Therefore, it is necessary to provide an atomizer and a control method for controlling the atomizer to solve the technical problems mentioned above.

BRIEF DESCRIPTION OF THE DRAWINGS

FIG. 1 is a schematic diagram of an atomizer according to an exemplary embodiment of the present disclosure.
FIG. 2 is a module diagram of the atomizer in Fig. 1.
FIG. 3 is a schematic diagram of an atomizer according to another exemplary embodiment of the present disclosure.
FIG. 4 is a cross-sectional view of an atomizer according to still another exemplary embodiment of the present disclosure.
FIG. 5 is a flow chart diagram of a control method of an atomizer according to an exemplary embodiment of the present disclosure.
FIG. 6 is a schematic diagram of an electronic cigarette according to an exemplary embodiment of the present disclosure.

DETAILED DESCRIPTION

For a thorough understanding of the present disclosure, numerous specific details are set forth in the following description for purposes of illustration but not of limitation, such as particularities of system structures, interfaces, techniques, et cetera. However, it should be appreciated by those of skill in the art that, in absence of these specific details, the present disclosure may also be carried out through other implementations. In other instances, a detailed description of well-known devices, circuits, and methods is omitted, so as to avoid unnecessary details from hindering the description of the disclosure.

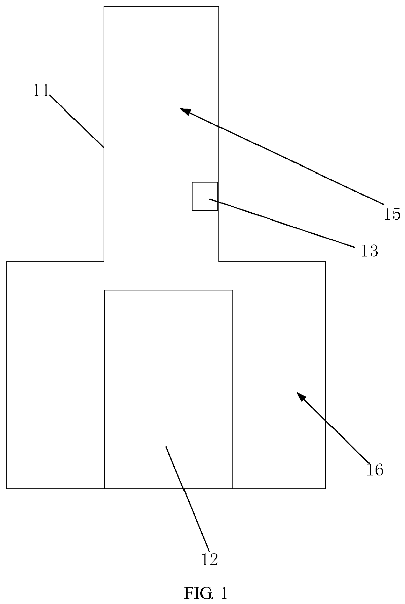

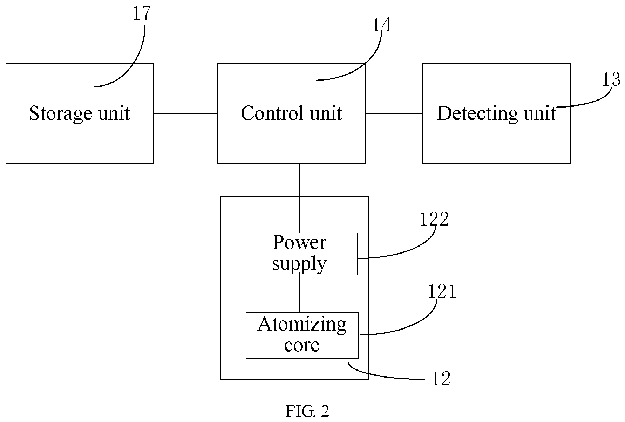

Referring to FIGS. 1 and 2, FIG. 1 is a schematic diagram of an atomizer according to an exemplary embodiment of the present disclosure. FIG. 2 is a module diagram of the atomizer in FIG. 1. In this embodiment, the atomizer may include a housing 11, an atomizing unit 12, a detecting unit 13 and a control unit 14.

The housing 11 may define a gas passage 15 and an accommodation space 16 communicated with the gas passage 15.

The atomizing unit 12 may be arranged in the accommodation space 16 and configured to generate aerosol passing through the gas passage 15 for user.

The detecting unit 13 may be arranged in the gas passage 15 and configured to detect a gas flow quantity in the gas passage 15. The detecting unit 13 may be arranged in any position in the gas passage 15, for example, the detecting unit 13 may be fixed on an inner wall of the gas passage 15 defined by the housing 11. In other embodiments, the detecting unit 13 may be arranged in the gas passage 15 in other manners.

In this embodiment, the detecting unit 13 may be a rate detecting unit configured to detect a gas flow rate in the gas passage 15 and calculate the gas flow quantity according to the gas flow rate. For instance, in an embodiment, the gas flow quantity Q could be calculated according to a cross section area S of a position of the gas passage 15 at which the detecting unit 13 is located, the fluid Bernoulli equation and the detected gas flow rate v. For example, in an ideal modeling, $Q = v \times S$. The cross section area S of a position of the gas passage 15 at which the detecting unit 13 is located may be a premeasured parameter.

In other embodiments, the detecting unit 13 may be a gas pressure detecting unit configured to detect a gas pressure in the gas passage 15 and calculate the gas flow quantity according to the gas pressure. Particularly, the gas flow quantity could be calculated according to gas pressure, and parameters of the position of the gas passage 15 at which the detecting unit 13 is located. For example, the gas flow quantity Q may be calculated according to gas density r, the cross section area S of the position of the gas passage 15 at which the detecting unit 13 is located, the gas pressure P of the position at which the detecting unit 13 is located and the fluid Bernoulli equation. For example, in an ideal modeling,

$$Q = S^2 \times \sqrt{2P/r}$$ . The cross section area S of the position of the gas passage 15 at which the detecting unit 13 is located may be a pre measured parameter. The gas density r may be a known parameter.

Without doubt, the gas flow quantity may be detected by other methods in other embodiments.

The control unit 14 may be electrically connected to the detecting unit 13 and the atomizing unit 12 and configured to adjust an atomizing quantity of the atomizer according to the gas flow quantity detected by the detecting unit 13.

Specifically, the atomizer may further include a storage unit 17 configured to store a preset mapping table between the gas flow quantity and the atomizing quantity. The control unit 14 may be electrically connected to the storage unit 17 and configured to find a corresponding atomizing quantity in the preset mapping table according to the gas flow quantity detected by the detecting unit 13 and adjust the atomizing quantity of the atomizer to the corresponding atomizing quantity.

In other embodiments, the atomizer may not include the storage unit 17. The control unit 14 may calculate a target atomizing quantity according to the detected gas flow quantity and adjust the atomizing quantity of the atomizer to the target atomizing quantity.

In other embodiments, the control unit 14 may calibrate the target atomizing quantity according to the corresponding atomizing quantity to obtain a calibrated atomizing quantity and adjust the atomizing quantity of the atomizer to the calibrated atomizing quantity.

Specifically, the atomizing unit 12 may include an atomizing core 121 and a power supply 122 supplying power to the atomizing core 121. The control unit 14 may be electrically connected to the power supply 122 and adjust the atomizing quantity of the atomizing unit 12 via adjusting an output power of the power supply 122, so as to realize the purpose of adjusting the atomizing quantity of the atomizer.

According to the above methods, when the atomizing quantity of the atomizer is adjusted via adjusting the output power of the power supply 122, there may be several cases as follows:

In the first case, the control unit 14 may calculate a target output power of the power supply 122 according to the detected gas flow quantity, and then adjust the output power of the power supply 122 to the target output power of the power supply 122 so as to adjust the atomizing quantity of the atomizer.

In the second case, the mapping table between the gas flow quantity and the atomizing quantity may be replaced with a mapping table between the gas flow quantity and the output power of the power supply 122. The control unit 14 may find a corresponding output power in the mapping table between the gas flow quantity and the output power of the power supply according the gas flow quantity detected by the detecting unit 13, and adjust the output power of the power supply 122 to the corresponding output power so as to adjust the atomizing quantity of the atomizer.

In the third case, the control unit 14 may calibrate the target output power according to the corresponding output power to obtain a calibrated output power, and then adjust the output power of the power supply 122 to the calibrated output power so as to adjust the atomizing quantity of the atomizer to the calibrated atomizing quantity.

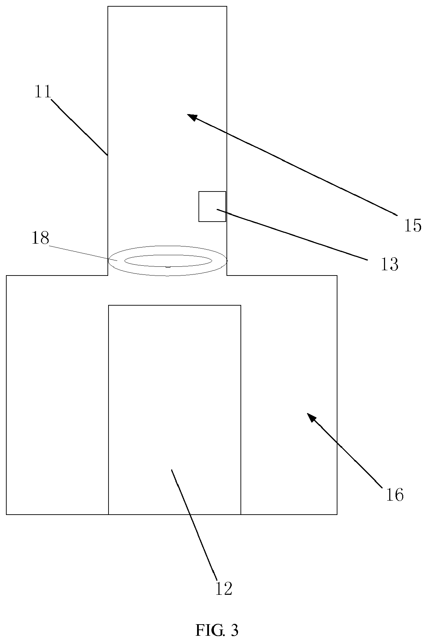

In other embodiments, the atomizing quantity may be adjusted by other methods. Referring to Fig. 3, an atomizer according to another exemplary embodiment of the present disclosure is depicted. In this embodiment, the atomizer may further include a throttle control unit 18 electrically connected to the control unit 14. The throttle control unit 18 may be arranged in the gas passage 15. The control unit 14 may control the atomizing quantity of the atomizer via controlling a gas flux of the throttle control unit 18. In this embodiment, the throttle control unit 18 may be arranged in a parting of the gas passage 15 and the accommodation space 16. Of course, in other embodiments, the throttle control unit 18 may be arranged in any position in the gas passage 15. The gas flux of the throttle control unit 18 may be controlled by the control unit 14, specifically, via changing an internal diameter of the throttle control unit 18 by the control unit 14. In some embodiments, the throttle control unit 18 may be an electronic control gas valve.

In other embodiments, the control unit 14 may adjust the atomizing quantity of the atomizer by adjusting the output power of the power supply as well as adjusting the gas flux of the throttle control unit.

Specifically, the atomizer may further include other parts, such as, a reservoir for storing tobacco liquid. The atomizer 12 may atomize the tobacco liquid absorbed from the reservoir to form aerosol which may pass through the gas passage 15 for user.

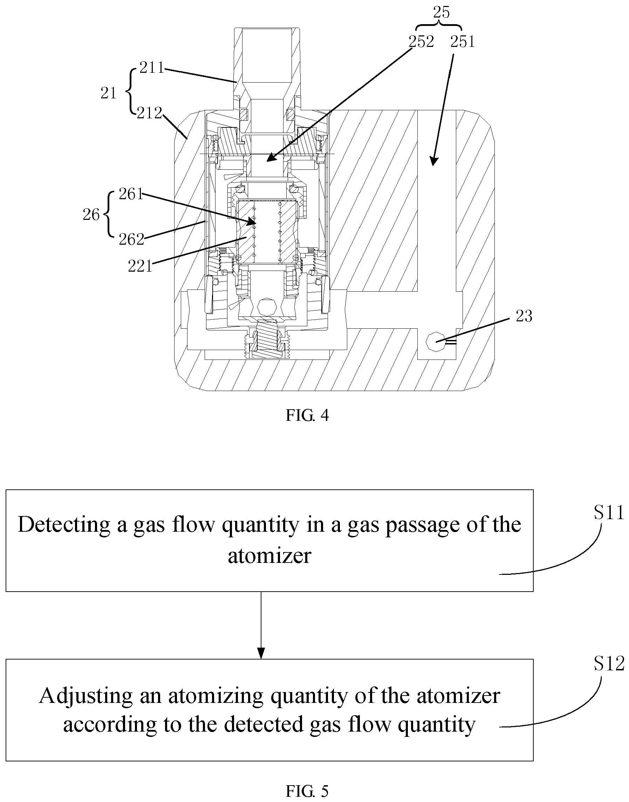

Please referring to FIG. 4, a cross-sectional view of an atomizer according to still another exemplary embodiment of the present disclosure is depicted. In this embodiment, the atomizer may include a housing 21, an atomizing unit (not labeled), a detecting unit 23 and a control unit (not labeled).

The electrically connections of the control unit, the atomizing unit and the detecting unit 23 are similar to the previous embodiments, which is not recited herein.

The housing 21 may define a gas passage 25 and an accommodation space 26 communicated with the gas passage 25.

The atomizing unit may be arranged in the accommodation space 26 and configured to generate aerosol passing through the gas passage 25 for user.

The detecting unit 23 may be arranged in the gas passage 25 and configured to detect a gas flow quantity in the gas passage 25. The detecting unit 23 may be arranged in any position in the gas passage 25, for example, the detecting unit 23 may be fixed on an inner wall of the gas passage 25 defined by the housing 21. In other embodiments, the detecting unit 23 may be arranged in the gas passage 25 in other manners.

The ways how to detect the gas flow quantity by the detecting unit 23 are similar to those of the detecting unit in the embodiments mentioned above, which are not recited herein.

The control unit is electrically connected to the detecting unit 23 and the atomizing unit, and is configured to adjust an atomizing quantity of the atomizer according to the gas flow quantity detected by the detecting unit 23.

The ways that the control unit adjusts the atomizing quantity of the atomizer may be similar to those of the previous embodiments, and are not recited herein.

Particularly, the atomizing unit may include an atomizing core 221 and a power supply (not labeled) providing power to the atomizing core 221. The control unit may be electrically connected to the power supply and adjust the output power of the power supply to adjust the atomizing quantity of the atomizing unit, so as to adjust the atomizing quantity of the atomizer. In other embodiments, the atomizing quantity of the atomizer could be adjusted via controlling the throttle control unit, which could be referred to the previous embodiments.

In this embodiment, the housing 21 may include a first sub-housing 211 and a second sub-housing 212. The gas passage 25 may include a gas inlet channel 251 and an aerosol outlet channel 252. The accommodation space 26 may include a first sub-accommodation space 261 and a second sub-accommodation space 262. The first sub-housing 211 may define the aerosol outlet channel 252 and the first sub-accommodation space 261 communicated with the aerosol outlet channel 252. The second sub-housing 212 may define the second sub-accommodation space 262 and the gas inlet channel 251 communicated with the first sub-accommodation space 261. The first sub-accommodation space 261 may accommodate the atomizing core 221. The second sub-accommodation space 262 may accommodate the power supply. The power supply may include a battery or a battery group and an output power regulation module. The control unit may be electrically connected to the output power regulation module to adjust the output power of the power supply.

In this embodiment, the detecting unit 23 may be arranged in the gas inlet channel 251 to improve the stability of detecting the gas flow quantity of the detection unit 23. The detecting unit 23 may be fixed on the inner wall of the gas inlet channel 251. It should be understood that, the detecting unit 23 may also be arranged in the aerosol outlet channel 252 or any position in the gas passage 25. In other embodiments, there may be a plurality of detecting units 23 arranged in different positions of the passage 25 respectively, for example, one detecting unit 23 may be arranged in the aerosol outlet channel 252, another one may be arranged in the gas inlet channel 251, and so on. The control unit may receive the gas flow quantity detected by the plurality of detection units 23 and then calculate an average value, so as to avoid the inaccuracy of the detection data caused by the difference and instability of detecting the gas flow quantity of the plurality of detection units 23 in the aerosol outlet channel 252 and the gas inlet channel 251.

Referring to Fig. 5, a flow chart diagram of a control method of an atomizer according to an exemplary embodiment of the present disclosure is depicted. In this embodiment, the control method of the atomizer may include:

S11: detecting a gas flow quantity in a gas passage of the atomizer.

In an embodiment, the block S11 may further include: detecting gas flow rate in the gas passage; and calculating the gas flow quantity according to the gas flow rate. For instance, in an embodiment, the gas flow quantity Q could be calculated according to a cross section area S of the gas passage 15 at which the detecting unit 13 is located, the fluid Bernoulli equation and the detected gas flow rate v. For example, in an ideal modeling, Q=v×S. The cross section area S of the gas passage 15 at which the detecting unit 13 is located may be a pre-measured parameter.

In another embodiment, the block S11 may further include: detecting gas pressure in the gas passage and calculating the gas flow quantity according to the gas pressure. Specifically, the gas flow quantity Q may be calculated according to gas density r, the cross section area S of the gas passage 15 at which the detecting unit 13 is located, the gas pressure P of the position at which the detecting unit 13 is located and the fluid Bernoulli equation. For example, in an ideal modeling,

$$Q = S^2 \times \sqrt{2P/r}$$

In other embodiments, the gas flow quantity may be detected via other ways.

S12: adjusting an atomizing quantity of the atomizer according to the detected gas flow quantity.

In this embodiment, the block S12 may include: finding a corresponding atomizing quantity in a preset mapping table between the gas flow quantity and the atomizing quantity according to the detected gas flow quantity, and adjusting the atomizing quantity to the corresponding atomizing quantity. The atomizing quantity may be adjusted to the corresponding atomizing quantity via adjusting the output power of the atomizing unit.

In one embodiment, the preset mapping table between the gas flow quantity and the atomizing quantity may be replaced with a preset mapping table between the gas flow quantity and the output power of the atomizing unit. The block S12 may include: finding a corresponding output power in the preset mapping table according to the detected gas flow quantity and adjusting the output power of the atomizing unit to the corresponding output power.

In another embodiment, the block S12 may further include: calculating a target output power of the atomizing unit according to the detected gas flow quantity, and adjusting the output power of the atomizing unit to the target output power, so as to adjust the atomizing quantity of the atomizer.

In other embodiments, the block S12 may further include: calibrating the target atomizing quantity according to the atomizing quantity to obtain a calibrated atomizing quantity, and adjusting the atomizing quantity to the calibrated atomizing quantity.

In other embodiments, the block S12 may further include: adjusting the atomizing quantity of the atomizer via adjusting the output power of the atomizing unit as well as the gas flux of the throttle control unit 18.

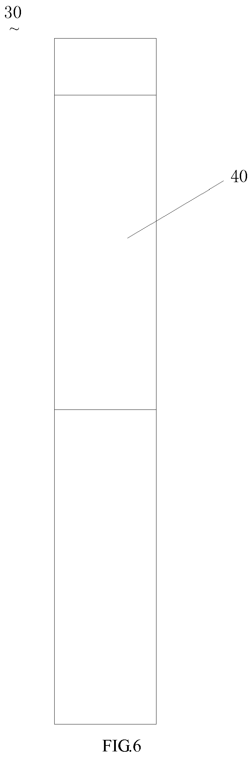

Referring to Fig. 6, an electronic cigarette according to an exemplary embodiment of the present disclosure is depicted. The electronic cigarette 30 of the present disclosure may include an atomizer 40, the atomizer 40 may be the atomizer in any embodiment mentioned above.

In the present disclosure, a detecting unit is arranged in the gas passage to detect the gas flow quantity, and then the control unit adjusts the atomizing quantity of the atomizer according to the gas flow quantity detected by the detecting unit, which could guide the user to smoke via using the electronic cigarette in a healthier way.

The above description depicts merely some exemplary embodiments of the disclosure, but is not meant to limit the scope of the disclosure. Any equivalent structure or flow transformations made to the disclosure, or any direct or indirect applications of the disclosure on other related fields, shall all be covered within the protection of the disclosure.

**Claims**

1. An atomizer, comprising:

   a housing (11, 21), defining a gas passage (15, 25) and an accommodation space (16, 26) communicated with the gas passage (15, 25);
   an atomizing unit (12), arranged in the accommodation space (16, 26) and configured to generate aerosol passing through the gas passage (15, 25) for user;
   it is **characterized in that** the atomizer further comprises:

      a detecting unit (13, 23), arranged in the gas passage (15, 25) and configured to detect a gas flow quantity in the gas passage (15, 25); and
      a control unit (14), electrically connected to the detecting unit (13, 23) and configured to adjust an atomizing quantity of the atomizer according to the gas flow quantity detected by the detecting unit (13, 23).

2. The atomizer of claim 1, wherein the detecting unit (13, 23) is a gas pressure detecting unit configured to detect a gas pressure in the gas passage (15, 25) and calculate the gas flow quantity according to the gas pressure.

3. The atomizer of claim 1, wherein the detecting unit (13, 23) is a rate detecting unit configured to detect a gas flow rate and calculate the gas flow quantity according to the gas flow rate.

4. The atomizer of claim 1, further comprising a storage unit (17) configured to store a preset mapping table between the gas flow quantity and the atomizing quantity;
   wherein the control unit (14) is further configured to:

      find a corresponding atomizing quantity in the preset mapping table according to the gas flow quantity detected by the detecting unit (13, 23); and
      adjust the atomizing quantity to the corresponding atomizing quantity.

5. The atomizer of claim 1, wherein the control unit (14) is further configured to calculate a target atomizing quantity according to the gas flow quantity and adjust the atomizing quantity of the atomizer to the target atomizing quantity.

6. The atomizer of claim 1, further comprising a storage unit (17) configured to store a preset mapping table between the gas flow quantity and the atomizing quantity;
   wherein the control unit (14) is further configured to:

      find a corresponding atomizing quantity in the preset mapping table according to the gas flow quantity detected by the detecting unit (13, 23);
      calculate a target atomizing quantity according to the gas flow quantity detected by the detecting unit (13, 23);
      calibrate the target atomizing quantity according to the corresponding atomizing quantity to obtain a calibrated atomizing quantity; and
      adjust the atomizing quantity of the atomizer to the calibrated atomizing quantity.

7. The atomizer of claim 1, wherein the atomizing unit further comprises a power supply and an atomizing core (221) electrically connected to power supply, the control unit is electrically connected to the power supply and configured to adjust the atomizing quantity of the atomizer via adjusting an output power of the power supply.

8. The atomizer of claim 1, further comprising a throttle control unit (18) arranged in the gas passage and electrically connected to the control unit, wherein the control unit is further configured to adjust the atomizing quantity of the atomizer via controlling a gas flux of the throttle control unit (18).

9. The atomizer of claim 1, further comprising a throttle control unit (18) arranged in the gas passage (15) and electrically connected to the control unit (14); wherein the atomizing unit (12) further comprises a power supply (122) and an atomizing core (121) electrically connected to power supply (122); the control unit (14) is electrically connected to the power supply (122) and configured to adjust the atomizing quantity of the atomizer via adjusting an output power of the power supply (122) and a gas flux of the throttle control unit (18).

10. The atomizer of claim 8 or claim 9, wherein the throttle control unit (18) is arranged in a parting of the gas passage (15) and the accommodation space (16).

11. The atomizer of claim 10, wherein the gas flux of the throttle control unit (18) is controlled via changing an internal diameter of the throttle control unit (18) by the control unit (14).

12. A control method of an atomizer, **characterized in that** the method comprises:

    detecting a gas flow quantity in a gas passage of the atomizer (S11); and
    adjusting an atomizing quantity of the atomizer according to the detected gas flow quantity (S12).

13. The control method of claim 12, wherein the detecting gas flow quantity in the gas passage of the atomizer (S11) comprises:

    detecting a gas pressure in the gas passage; and
    calculating the gas flow quantity according to the gas pressure.

14. The control method of claim 12, wherein the detecting gas flow quantity in the gas passage of the atomizer (S11) comprises:

    detecting a gas flow rate in the gas passage; and
    calculating the gas flow quantity according to the gas flow rate.

15. An electronic cigarette, comprising an atomizer (40), wherein the atomizer (40) comprises:

    a housing (11, 21), defining a gas passage (15, 25) and an accommodation space (16, 26) communicated with the gas passage (15, 25);
    an atomizing unit (12), arranged in the accommodation space (16, 26) and configured to generate aerosol passing through the gas passage (15, 25) for user;
    it is **characterized in that** the atomizer further comprises:

        a detecting unit (13, 23), arranged in the gas passage (15, 25) and configured to detect a gas flow quantity in the gas passage (15, 25); and
        a control unit (14), connected to the detecting unit (13, 23) and configured to adjust an atomizing quantity of the atomizer according to the gas flow quantity detected by the detecting unit (13, 23).

11

15

13

16

12

FIG. 1

FIG. 2

FIG. 3

FIG. 4

Detecting a gas flow quantity in a gas passage of the atomizer — S11

Adjusting an atomizing quantity of the atomizer according to the detected gas flow quantity — S12

FIG. 5

30

~

40

FIG.6

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 201710034320